# DEMANDE DE BREVET EUROPEEN

(11) **EP 0 798 011 A1**
(43) Date de publication de la demande: **01.10.1997**
(21) Numéro de dépôt: 97400642.1
(22) Date de dépôt: 21.03.1997
(51) Int. Cl.: A61M 27/00

(54) **Valve de drainage implantable pour le traitement de l'hydrocéphalie**

(30) Priorité: 26.03.1996 FR 9603721
(71) Demandeur: CORDIS S.A., F-91170 Viry-Châtillon (FR)
(72) Inventeur: Bonnal, Olivier, 06860 Cagnes sur Mer (FR)
(74) Mandataire: Geismar, Thierry

(57) **Abrégé**

L'invention concerne une valve de drainage implantable pour le traitement de l'hydrocéphalie, comprenant un corps (1) formant un siège (3) de valve, un organe de fermeture (4), un ressort (5) agencé pour appliquer l'organe de fermeture sur le sigèe, et une masselotte (6, 6', 6") de compensation des différences de hauteur de colonne de liquide en fonction de la position du patient sur lequel la valve est implantée.

Selon l'invention, ladite masselotte est disposée entre ledit organe de fermeture et une extrémité du ressort dont l'autre extrémité est en appui sur un organe de butée (7).

## Description

La présente invention concerne une valve de drainage implantable pour le traitement de l'hydrocéphalie, et plus particulièrement une telle valve du type comprenant un corps formant un siège de valve, un organe de fermeture, un ressort agencé pour appliquer l'organe de fermeture sur le siège, et une masselote de compensation des différences de hauteur de colonne de liquide en fonction de la position du patient porteur de la valve.

On connaît déjà divers types de valves destinées à contrôler le drainage d'un excès de fluide céphalo-rachidien contenu dans les ventricules cérébraux vers, par exemple, la cavité péritonéale.

C'est ainsi que le document EP-A-0617975 décrit une telle valve comprenant un corps formant un siège de valve, un organe de fermeture tel qu'une bille, et un ressort dont une extrémité applique l'organe de fermeture sur le corps, donc une valve du type clapet. Dans ce document, cette valve comprend en outre une masselotte sur laquelle s'appuie l'extrémité du ressort opposée à l'organe de fermeture.

Une telle valve permet de tenir compte des différences de hauteur de colonne de liquide en fonction de la position, notamment assise ou couchée, du patient. En effet, lorsque la valve est en position horizontale, la bille n'est soumise qu'à la pression différentielle entre l'amont et l'aval et à la force exercée par le ressort. Au contraire, en position verticale, la masselotte dont le poids est supérieur à la force du ressort, vient légèrement comprimer ce dernier, et son poids vient se substituer à la force du ressort pour plaquer la bille sur son siège, avec donc une force supérieure.

Bien qu'elle donne d'une manière générale satisfaction, un inconvénient de cette valve réside dans le fait qu'en position verticale, la masselotte peut se déplacer dans une certaine mesure dans son logement. Il peut en résulter des effets dynamiques, ou balistiques, indésirables lors de mouvements saccadés tels que la course, effets dynamiques qui peuvent perturber le bon fonctionnement du dispositif.

La présente invention vise à pallier ces inconvénients.

A cet effet, l'invention a pour objet une valve de drainage implantable pour le traitement de l'hydrocéphalie, comprenant un corps formant un siège de valve, un organe de fermeture, un ressort agencé pour appliquer l'organe de fermeture sur le siège, et une masselotte de compensation des différences de hauteur de colonne de liquide en fonction de la position du patient sur lequel la valve est implantée, caractérisée par le fait que ladite masselotte est disposée entre ledit organe de fermeture et une extrémité du ressort dont l'autre extrémité est en appui sur un organe de butée.

Par conséquent, le ressort agit sur l'organe de fermeture par l'intermédiaire de la masselotte. La valve est implantée sur le patient de manière que, lorsque ce dernier est en position couchée, la masselotte ne soit pas supportée par l'organe de fermeture et que donc seule la force du ressort s'exerce sur l'organe de fermeture. Au contraire, lorsque le patient est en position debout, c'est l'organe de fermeture qui supporte la masselotte dont le poids vient s'ajouter à la force du ressort, compensant ainsi la hauteur de colonne de liquide correspondant à la station debout.

Dans un mode de réalisation particulier de l'invention, ladite autre extrémité du ressort est en appui sur un organe solidaire du corps ou, bien évidemment, de manière équivalente, sur le corps lui-même.

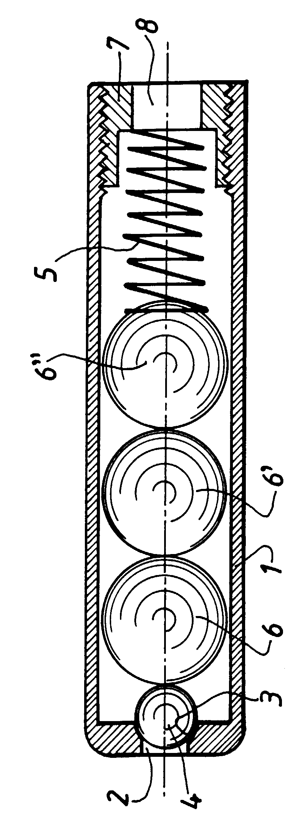

On décrira maintenant, à titre d'exemple non limitatif, un mode de réalisation particulier de l'invention, en référence à la figure unique annexée représentant une vue en coupe axiale d'une valve selon l'invention.

Cette valve comporte un corps cylindrique tubulaire 1 ouvert à ses deux extrémités. La périphérie de l'ouverture amont 2 forme un siège conique 3 sur lequel une bille de fermeture 4 est appliquée par un ressort hélicoïdal 5 par l'intermédiaire de trois billes 6, 6' et 6" formant masselotte.

La bille masselotte 6 est appliquée sur la bille de fermeture 2, la bille 6' sur la bille 6, et la bille 6" sur la bille 6'. Une des extrémités du ressort est en appui sur la bille 6" et son autre extrémité est en appui sur une vis annulaire 7, maintenant l'ensemble des éléments précités à l'intérieur du corps tubulaire 1 et formant en son centre l'ouverture aval 8 de la valve.

En utilisation, l'ouverture amont 2 est raccordée à un cathéter dont l'extrémité libre est placée dans le ventricule cérébral à drainer, et l'ouverture aval 8 est raccordée à un cathéter de drainage dont l'extrémité libre est ramenée dans la cavité péritonéale.

La valve est implantée sur le patient de manière que, lorsque ce dernier est debout, son axe soit sensiblement vertical. La bille 2 subit alors un effort vertical de haut en bas, l'appliquant sur le siège 3, égal à la somme de la force exercée par le ressort et du poids des billes 6, 6' et 6".

Au contraire, lorsque le patient est couché, les billes 6, 6' et 6" sont supportées par la paroi intérieure du corps 1, de sorte que la bille 2 ne subit que la force du ressort.

## Revendications

1. Valve de drainage implantable pour le traitement de l'hydrocéphalie, comprenant un corps (1) formant un siège (3) de valve, un organe de fermeture (4), un ressort (5) agencé pour appliquer l'organe de fermeture sur le siège, et une masselotte (6, 6', 6") de compensation des différences de hauteur de colonne de liquide en fonction de la position du patient sur lequel la valve est implantée, caractérisée par le fait que ladite masselotte est disposée entre ledit organe de fermeture et une extrémité du ressort dont l'autre extrémité est en appui sur un organe de butée (7).

2. Valve selon la revendication 1, dans laquelle ladite autre extrémité du ressort est en appui sur un organe solidaire du corps.
